# EUROPEAN PATENT APPLICATION

(11) **EP 3 117 823 A1**
(43) Date of publication of application: **18.01.2017**
(21) Application number: 15382367.9
(22) Date of filing: 17.07.2015
(51) Int. Cl.: A61K 9/19, C07D 257/04, C07C 233/47

(54) **AMORPHOUS SOLID DISPERSION COMPRISING AN ANGIOTENSIN RECEPTOR BLOCKER AND A NEUTRAL ENDOPEPTIDASE INHIBITOR**

(71) Applicant: Quimica Sintetica, S.A., 08028 Barcelona (ES)
(72) Inventor: BARRECA, Giuseppe, 23874 MONTEVECCHIA (IT); VENTIMIGLIA, Giampiero, 72021 FRANCAVILLA FONTANA (IT); BELLOMI, Sonja, 28100 NOVARA (IT)
(74) Representative: Oficina Ponti, SLP

(57) **Abstract**

Are described an amorphous solid dispersion comprising a 1:1 stoichiometric mixture of the sodium salts of Valsartan and Sacubitril useful in the treatment of essential hypertension and/or cardiac failure, possibly in the form of a pharmaceutical composition comprising said amorphous solid dispersion and at least one pharmaceutically acceptable carrier, as well as methods for the preparation of the amorphous solid dispersion.

## Description

### Field of the Invention

The present invention relates to an amorphous solid dispersion comprising an angiotensin receptor blocker and a neutral endopeptidase inhibitor, a pharmaceutical composition comprising said solid dispersion as well as the process for obtaining the same.

### State of the Art

Angiotensin II is a hormone that causes blood vessel to constrict. This, in turn can result in high blood pressure and strain on the heart. It is known that angiotensin II interacts with specific receptors on the surface of target cells. Two receptor subtypes for angiotensin II, namely AT1 and AT2, have been identified so far. In recent times, great efforts have been made to identify substances that bind to the AT1 receptor. Angiotensin receptor blockers (ARBs, angiotensin II antagonists) are now known to prevent angiotensin II from binding to its receptors in the walls of blood vessels, thereby resulting in lower blood pressure. Because of the inhibition of the AT1 receptor, such antagonists can be used, therefore, as anti-hypertensives or for the treatment of congestive heart failure, among other indications.

Neutral endopeptidase (NEP) is a zinc-containing metalloprotease that cleaves a variety of peptide substrates on the amino side of hydrophobic residues. Substrates for this enzyme include, but are not limited to, atrial natriuretic peptide (ANP, also known as ANF), brain natriuretic peptide (BNP), met- and leu-enkephalin, bradykinin, neurokinin A, endothelin-1 and substance P. ANP is a potent vasorelaxant and natriuretic agent. Infusion of ANP in normal subjects resulted in a reproducible, marked enhancement of natriuresis and diuresis, including increases in fractional excretion of sodium, urinary flow rate and glomerular filtration rate. However, ANP has a short half-life in circulation, and NEP in kidney cortex membranes has been shown to be the major enzyme responsible for degrading this peptide. Thus, inhibitors of NEP (neutral endopeptidase inhibitors, NEPi) should increase plasma levels of ANP and, hence, are expected to induce natriuretic and diuretic effects.

While substances, such as angiotensin receptor blockers and neutral endopeptidase inhibitors may be useful in the control of hypertension, essential hypertension is a polygenic disease and is not always controlled adequately by monotherapy. Approximately 333 million adults in economically developed countries and about 65 million Americans (1 in 3 adults) had high blood pressure in 2000. Prolonged and uncontrolled hypertensive vascular disease ultimately leads to a variety of pathological changes in target organs, such as the heart and kidney. Sustained hypertension can lead as well to an increased occurrence of stroke.

A supramolecular complex of two active agents with different mechanisms of action, namely an angiotensin receptor antagonist and a neutral endopeptidase inhibitor dual-acting compound useful for the treatment of patients with various cardiovascular and/or renal diseases has been first disclosed in the International patent application. WO 2007/056546 A1.

According to the description of WO 2007/056546, the angiotensin receptor blocker is preferably Valsartan (depicted below) (also known as ((*S*)-*N*-valeryl-*N*-{[2'-(1*H*-tetrazole-5-yl)-biphenyl-4-yl]-methyl}-valine), while the neutral endopeptidase inhibitor is preferably Sacubitril (depicted below) (i.e. (2*R*,4*S*)-5-biphenyl-4-yl-5-(3-carboxy-propionylamino)-2-methyl-pentanoic acid ethyl ester).

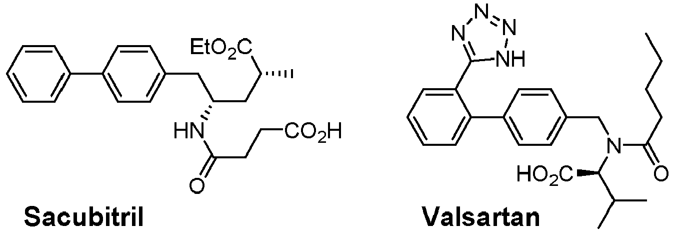

A particularly useful therapeutic agent is the supramolecular complex, trisodium [3-((1*S*,3*R*)-1-biphenyl-4-yl-methyl-3-ethoxycarbonyl-1-butylcarbamoyl)propionate-(*S*)-3'-methyl-2'-(pentanoyl{2"-(tetrazol-5-ylate)biphenyl-4'-yl-methyl}amino)butyrate]-hemi-pentahydrate, generally referred to as LCZ-696.

The formulation of dual acting compounds such as supramolecular complexes is not trivial since typical formulation techniques may have a negative effect on the drug substance leading to e.g. increased amorphism and/or dissociation of the components of the dual acting compound. In general, one should avoid exposing the therapeutic agent during the formulation to moisture, excessive heat and/or high shear forces. This may pose a number of formulation issues and difficulties, which need to be addressed.

An object of the present invention is, therefore, to provide an amorphous solid dispersion containing an angiotensin receptor blocker and a neutral endopeptidase inhibitor, as well as a pharmaceutical composition comprising the same, which are stable and pure in the amorphous state upon storage under stress conditions and that are obtainable in an easy and reliable manner, e.g. even in large scale.

### Summary of the invention

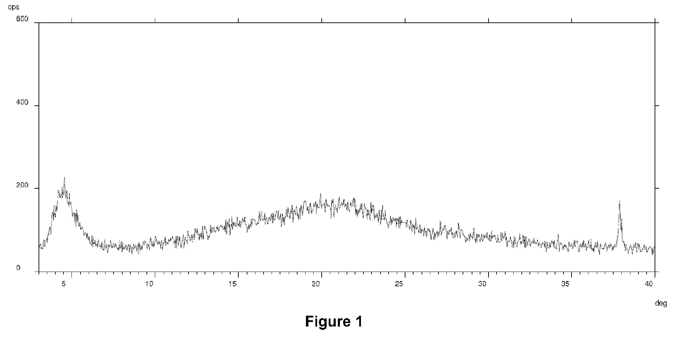

These objects are achieved with the present invention, which in its first aspect relates to an amorphous solid dispersion comprising a 1:1 stoichiometric mixture of the sodium salts of Valsartan and Sacubitril. The amorphous solid dispersion is characterized by XRPD substantially as illustrated by figure 1.

In a second aspect thereof, the present invention relates to methods for the preparation of the amorphous solid dispersion described above.

A first embodiment of the second aspect of the invention consists in a method for the preparation of the amorphous solid dispersion described before including the following steps:
a) dissolving LCZ-696 in water;
b) freeze-drying the aqueous solution obtained in step a).

A second embodiment of this aspect of the invention consists in a method for the preparation of said amorphous solid dispersion including the following steps:
c) mixing a 1:1 stoichiometric mixture of Valsartan and Sacubitril with water, a water miscible solvent or a mixture thereof wherein the volume ratio between water and water miscible solvent is comprised between 80:20 and 50:50;
d) adding sodium hydroxide in a ratio of 3:1 mole/mole with respect to the 1:1 stoichiometric mixture of Valsartan and Sacubitril;
e) freeze-drying the solution obtained in step d).

In a possible variant of this embodiment, between steps d) and e) a further step d') is carried out, consisting of adjusting the pH of the mass obtained in step d) between 9.05 and 9.25; moreover, depending on the operating conditions (as described below), this second embodiment can optionally comprise, between steps d) and e), a step d") comprising distilling off the water miscible solvent and, if necessary, dilute the mass with water to obtain a solution. Steps d') and d"), if carried out both, can be carried out in any order.

In a third aspect the present invention relates to a pharmaceutical composition comprising said amorphous solid dispersion and at least one pharmaceutically acceptable carrier, as well as its use in the treatment of essential hypertension and/or cardiac failure.

### Brief Description of the Figures

Figure 1 shows a XRPD spectrum of the amorphous solid dispersion.

### Detailed Description of the Invention

All terms used in this application, unless otherwise specified, are to be understood in their ordinary meaning as known in the technical field. Other more specific definitions of certain terms used in this application are listed below and are intended to be applied uniformly to the entire application, unless otherwise indicated.

The term "about" includes the range of experimental error, which can normally occur when performing a measurement.

The term "mass" defines the combination of substrates, reagents, solvents, and products on which a physical or chemical transformation is carried out, and its meaning in the context of the present description and claims is further detailed below.

The term "excipient" means any substance contained in the final pharmaceutical form other than the active ingredient and which generally may not be therapeutically effective by itself. Excipients are essential for the administration of the active substance, as they allow to deliver the drug to the target site. Excipients are commonly referred to as raw materials entering into the composition of a pharmaceutical preparation with the aim of giving a shape, to facilitate administration and preserve the active ingredient. Furthermore, they contribute to characterize the pharmaceutical preparation from the point of view of appearance, stability, biopharmaceutical profile and acceptability by the patient.

X-ray powder diffractometry (XRPD) was used to characterize the amorphous solid dispersion obtained by the processes described in this application.

In general, the term "solid dispersion" refers to a system in a solid state (as opposed to a liquid or gaseous state) comprising at least two components, wherein one component is dispersed throughout the other component or components.

Preferred solid dispersions are "solid solutions", where the dispersion of the components is such that the system is chemically and physically uniform or homogeneous throughout or even consists of one phase as defined by measurement of thermodynamic properties of the system.

Amorphous solids generally possess crystal-like short range molecular arrangement, but no long range order of molecular packing as found in crystalline solids.

"Stable in the amorphous state upon storage under stress conditions" in the context of the present invention means that the solid dispersion of the present invention, when stored at a relative humidity of 50% at 25 °C for 24 hours, shows no signs of crystallinity associated to the sodium salts of Valsartan and/or Sacubitril or signs of cristallinity associated to LCZ-696 as judged by the absence of peaks in an X-ray powder diffractogram (XRPD).

By "chemically stable" it is meant that the amorphous solid dispersion of the present invention shows no degradation upon storage under stress conditions, i.e. when stored at 80 °C under *vacuum* for 24 hours. No degradation means that an HPLC analysis of Valsartan and Sacubitril shows no impurity of more than 0.1 area%.

A first aspect of the present invention relates to an amorphous solid dispersion comprising a 1:1 stoichiometric mixture of the sodium salts of Valsartan and Sacubitril characterized by a XRPD profile substantially as shown in figure 1. Preferably said amorphous solid dispersion comprises a 1:1 stoichiometric mixture of a disodium salt of Valsartan and a sodium salt of Sacubitril.

A second aspect of the present invention relates to two alternative methods for the preparation of said amorphous solid dispersion.

A first method includes steps a) and b).

In step a) LCZ-696 is dissolved in water at a temperature preferably comprised between 10 e 30 °C (e.g. between 20 and 25 °C). The amount of water can vary in a very wide range; preferably, its volume may vary between 5 mL and 15 mL per gram of LCZ-696 used; even more preferably, the volume is 10 mL per gram of LCZ-696.

In the subsequent step b) the solution obtained in step a) is freeze dried (lyophilized) according to the methods known in the field, comprising in general the freezing of a solution followed by a reduction of the pressure to remove the solvent. Conditions suitable for freezing the solutions of the invention may entail temperatures ranging between -80 °C and 20 °C (preferably -40 °C) at pressures preferably comprised between 130 and 110 mbar. The removal the solvent from the frozen solution may require temperatures generally lower than 20 °C (preferably 0 °C) at pressures of preferably 1 mbar.

A second embodiment of the second aspect of the present invention relates to a process for the preparation of the 1:1 stoichiometric mixture of the sodium salts of Valsartan and Sacubitril including steps c) to e).

In step c), a 1:1 stoichiometric mixture of Valsartan and Sacubitril is mixed with water, a water miscible solvent or a mixture thereof wherein the volume ratio between water and the organic solvent is comprised between 80:20 and 50:50, at a temperature preferably comprised between 10 e 30 °C (e.g. between 20 and 25 °C). The amount of water, of water miscible solvent or of the mixture thereof can vary in a very wide range; preferably, its volume may vary between 5 mL and 15 mL per gram of 1:1 stoichiometric mixture of Valsartan and Sacubitril used; even more preferably, the volume is 10 mL per gram of said 1:1 stoichiometric mixture.

Water miscible solvents suitable for the purpose are known and normally used in the field, such as, for example, a C1-C4 alcohol (for example ethanol, *tert*-butanol or preferably methanol) or C3-C6 ketone (e.g. acetone).

The subsequent step d) comprises the addition of sodium hydroxide in a ratio of 3:1 mole/mole with respect to the 1:1 stoichiometric mixture of Valsartan and Sacubitril.

Sodium hydroxide can be added in the form of an aqueous solution in case the previous step was performed in a water miscible solvent alone; alternatively, sodium hydroxide can be added in solid (powder) form. Depending on the way of addition of NaOH, the resulting mixture may be, in turn, either a solution or a wet solid-containing mixture; for this reason, the result of NaOH addition is referred to in this description and in the claims generally as "mass", which is intended to also include the case that the resulting mixture be a solution.

If the preceding steps, and in particular the addition of sodium hydroxide, have been carried out correctly, the resulting mass should have a pH value between 9.05 and 9.25, preferably between 9.15 and 9.20. If, at a control, it is observed that the pH of the mass lies outside this range, it is an indication that the stoichiometric ratio between the components of the mixture is not correct; in that case, a variant of the process object of the second embodiment of the invention includes an additional step d') wherein said pH value is adjusted between 9.05 and 9.25 by adding sodium hydroxide (if it is pH < 9.05) or a solution of the 1:1 stoichiometric mixture of Valsartan and Sacubitril (if it is pH > 9.25).

The last step e) comprises the lyophilization of the solution prepared in step d), after having possibly adjusted the pH of the mass in step d') using one of the procedure generally known in the field, for example those reported above to perform step b).

In the case when the water miscible solvent used in step c) is not compatible with the freeze-drying performed in step e), a variant of the process subject of the second embodiment of the present invention includes an additional step d"), carried out after either step d) or d'), in which said solvent (for example methanol) is evaporated under reduced pressure. If necessary, the mass obtained after distillation of the organic solvent is diluted with water to obtain a solution which can be lyophilized.

According to a third aspect of the present invention, the amorphous solid dispersion comprising a 1:1 stoichiometric mixture of the sodium salts of Valsartan and Sacubitril can be used, in mixture with one or more pharmaceutically acceptable excipients, for the preparation of pharmaceutical compositions useful in the treatment of essential hypertension and/or cardiac failure.

The present invention will be further illustrated through the following examples.

XRPD analyses were carried out with a diffractometer APD 2000 Ital Structures operating at room temperature, using as a source of X-ray a CuKα tube (40 kV, 30 mA, λ = 1.5406 Å). Acquisition was performed in step scan mode and in the Bragg-Brentano geometry, with steps of 0.04° per second over an interval 3-40° in theta/2theta. The samples were thoroughly ground in a mortar and placed in the recess of the aluminum sample holder. Before performing the measurement (remotely with WinAcq32 software), the instrument was calibrated with zinc oxide (purity > 99.5%).

The water content of the amorphous solid dispersions comprising a 1:1 stoichiometric mixture of the sodium salts of Valsartan and Sacubitril was determined by Karl Fischer analysis by means of a Mettler-Toledo DL38 automatic titrator, using Combititrant-5 as titrating medium (1 ml correspond to 5 mg of water) and Hydranal Ketosolver as a titration solvent.

**Freeze-drying cycle was performed on a preliminary lab scale by means of a Martin-Christ GmbH freeze-dryer, according to the following program:**

| **Step** | **Time** | **Pressure (mbar)** | **Temperature** |
|---|---|---|---|
| **1** | 3 hours | 130-110 | -40 °C |
| **2** | 15 hours | 1 | 0 °C |
| **3** | 3 hours | 1 | 10 °C |
| **4** | 20 hours | 1 | 15-20 °C |
| **5** | 3 hours | Up to atmospheric pressure | 25 °C |

### Example 1

### Preparation of an amorphous solid dispersion comprising a 1:1 stoichiometric mixture of the sodium salts of Valsartan and Sacubitril

LCZ-696 (1.0 g) (prepared according to the procedure described in example 1 of international application WO 2007/056546) was dissolved in water (10 mL) under magnetic stirring and at 25 °C. The obtained solution was freeze-dried to obtain an amorphous solid (final water content - as per Karl Fisher titration - 9.91% w/w) characterized by an XRPD spectrum as depicted in Figure 1.

### Example 2

### Preparation of an amorphous solid dispersion comprising a 1:1 stoichiometric mixture of the sodium salts of Valsartan and Sacubitril

Sacubitril (4.2 g, 10.2 mmol) and Valsartan (4.4 g, 10.2 mmol) were dissolved, under magnetic stirring and at 25 °C, in a 1:1 (vol/vol) mixture of methanol/water (80 mL). Sodium hydroxide was added (1.2 g, 30.6 mmol) checking the pH of the obtained solution (value comprised between 9.15 and 9.20). Methanol was stripped off under reduced pressure and water (30 mL) was added. The obtained solution was freeze-dried to obtain an amorphous solid characterized by an XRPD spectrum corresponding to the one obtained in example 1.

### Example 3

### Analysis of the thermal stability of the amorphous solid dispersion comprising a 1:1 stoichiometric mixture of the sodium salts of Valsartan and Sacubitril prepared according to the processes of the invention

The amorphous solid dispersion comprising a 1:1 stoichiometric mixture of the sodium salts of Valsartan and Sacubitril (10.0 g), prepared as described in Example 1, was maintained at 80 °C under *vacuum* for 24 hours. The solid was then brought to room temperature and subjected to XRPD analysis, giving rise to a XRPD spectrum corresponding to the one obtained in example 1 (final water content - as per Karl Fisher titration - 0.04% w/w).

### Example 4

### Analysis of the moisture stability of the amorphous solid dispersion comprising a 1:1 stoichiometric mixture of the sodium salts of Valsartan and Sacubitril prepared according to the processes of the invention

The amorphous solid dispersion comprising a 1:1 stoichiometric mixture of the sodium salts of Valsartan and Sacubitril (10.0 g), prepared as described in Example 1, was placed at 20-25 °C in a hydration chamber containing a saturated solution of potassium carbonate for 24 hours (50% ± 5% RH). The solid was then subjected to XRPD analysis, giving rise to a XRPD spectrum corresponding to the one obtained in example 1 (final water content - as per Karl Fisher titration - 11.75% w/w).

## Claims

1. An amorphous solid dispersion comprising a 1:1 stoichiometric mixture of the sodium salts of Valsartan and Sacubitril.

2. Amorphous solid dispersion according to claim 1 comprising a disodium salt Valsartan and a sodium salt of Sacubitril.

3. Method for the preparation of the amorphous solid dispersion of claims 1 and 2, including the steps of:
a) dissolving in water the supramolecular complex trisodium [3-((1S,3R)-1-biphenyl-4-yl-methyl-3-ethoxycarbonyl-1-butylcarbamoyl)propionate-(S)-3'-methyl-2'-(pentanoyl{2"-(tetrazol-5-ylate)biphenyl-4'-yl-methyl}amino)butyrate]-hemi-pentahydrate;
b) freeze-drying the aqueous solution obtained in step a).

4. Method according to claim 3, in which step a) is carried out at a temperature between 10 e 30 °C using a volume of water between 5 mL and 15 mL per gram of said supramolecular complex.

5. Method for the preparation of the amorphous solid dispersion of claims 1 and 2, including the steps of:
c) mixing a 1:1 stoichiometric mixture of Valsartan and Sacubitril with water, a water miscible solvent or a mixture thereof wherein the volume ratio between water and water miscible solvent is comprised between 80:20 and 50:50;
d) adding sodium hydroxide in a ratio of 3:1 mole/mole with respect to the 1:1 stoichiometric mixture of Valsartan and Sacubitril;
e) freeze-drying the solution obtained in step d).

6. Method according to claim 5 which, if the mass obtained on completion of step d) has a pH value not comprised between 9.05 and 9.25, includes an additional step d') between steps d) and e) wherein the pH value is adjusted in said range.

7. Method according to claim 5 which, if in step c) a water miscible solvent is used which is not compatible with the freeze-drying of step e), comprises an additional step d") between steps d) and e) in which said solvent is evaporated under reduced pressure and the thus obtained mass is diluted with water.

8. Method according to any one of claims 6 and 7, in which both the steps d') and d") are carried out.

9. Method according to any one of claims 5 to 8, in which step c) is carried out at a temperature between 10 e 30 °C using a volume of solvent between 5 mL and 15 mL per gram of said stoichiometric mixture of Valsartan and Sacubitril.

10. Method according to any one of claims 5 to 9, in which said water miscible solvent is selected among a C1-C4 alcohol and a C3-C6 ketone.

11. Method according to any one of claims 5 to 10, in which in step d) sodium hydroxide is added to the mass prepared in step c) in the form of an aqueous solution or in solid form.

12. Pharmaceutical compositions for the treatment of cardiac failure and/or essential hypertension comprising an amorphous solid dispersion according to any one of claims 1 and 2 in mixture with one or more pharmaceutically acceptable excipients and/or carriers.
